# FASCICULE DE BREVET EUROPEEN

(11) **EP 0 885 208 B1**
(45) Date de publication et mention de la délivrance du brevet: **16.08.2001**
(21) Numéro de dépôt: 97908304.5
(22) Date de dépôt: 05.03.1997
(51) Int. Cl.: C07D 305/14, A61K 31/335

(54) **TAXOIDES, LEUR PREPARATION ET LES COMPOSITIONS PHARMACEUTIQUES QUI LES CONTIENNENT**
TAXOIDE, IHRE HERSTELLUNG UND DIESE ENTHALTENDE PHARMAZEUTISCHE ZUSAMMENSETZUNGEN
TAXOIDS, THEIR PREPARATION AND PHARMACEUTICAL COMPOSITIONS CONTAINING THEM

(30) Priorité: 06.03.1996 FR 9602804
(43) Date de publication de la demande: 23.12.1998
(73) Titulaire: Aventis Pharma S.A., 92160 Antony (FR)
(72) Inventeur: BOUCHARD, Hervé, F-94320 Thiais (FR); BOURZAT, Jean-Dominique, F-94300 Vincennes (FR); COMMERCON, Alain, F-94400 Vitry-sur-Seine (FR)
(86) Numéro de dépôt international: FR9700386
(87) Numéro de publication internationale: WO9732869

(56) Documents cités:
- EP-A- 0 428 376
- EP-A- 0 555 485
- EP-A- 0 590 267
- EP-A- 0 694 539
- WO-A-94/21250
- WO-A-95/11241
- WO-A-96/03394
- CHEMICAL ABSTRACTS, vol. 119, no. 23, 6 Décembre 1993 Columbus, Ohio, US; abstract no. 240982r, XP002018843 & NATURE, vol. 364, no. 6436, 1993, LONDON GB, pages 464-466, K.C. NICOLAOU ET AL.:

## Description

La présente invention concerne de nouveaux taxoïdes de formule générale : 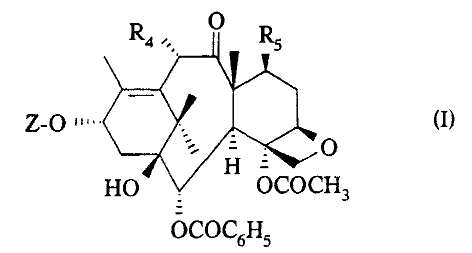 dans laquelle
Z représente un atome d'hydrogène ou un radical de formule générale : 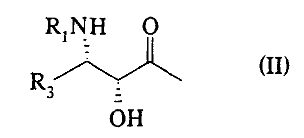 dans laquelle :
   R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente
      - un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
      - un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
      - ou un radical hétérocyclyle saturé contenant 4 à 6 atomes de carbone éventuellement substitué par un ou plusieurs radicaux alcoyles contenant 1 à 4 atomes de carbone,
   R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
   R₄ représente un radical hydroxy ou un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 2 à 6 atomes de carbone en chaîne droite ou ramifiée, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical carbamoyloxy, N-alcoylcarbamoyloxy dont la partie alcoyle contient 1 à 4 atomes de carbone, N,N-dialcoylcarbamoyloxy dont chaque partie alcoyle contient 1 à 4 atomes de carbone, benzoyloxy ou hétérocyclylcarbonyloxy dans lequel la partie hétérocyclique représente un hétérocycle aromatique à 5 ou 6 chaînons contenant un ou plusieurs hétéroatomes choisis parmi les atomes d'oxygène, de soufre ou d'azote,
   R₅ représente un radical alcoxy contenant 1 à 6 atomes ce carbone en chaîne droite ou ramifiée éventuellement substitué par un radical alcoxy contenant 1 à 4 atomes de carbone, alcényloxy contenant 3 à 6 atomes de carbone, alcynyloxy contenant 3 à 6 atomes de carbone, cycloalcoyloxy contenant 3 à 6 atomes de carbone, cycloalcényloxy contenant 3 à 6 atomes de carbone, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone.

De préférence les radicaux aryles pouvant être représentés par R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluoro-méthyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R₃ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

De préférence le radical R₄ représente un radical hydroxy ou un radical alcoxy droit ou ramifié contenant 1 à 6 atomes de carbone ou un radical alcanoyloxy contenanr 2 à 6 atomes de carbone éventuellement substitué par un radical méthoxy, éthoxy, méthylthio, éthylthio, carboxy, méthoxycarbonyle, éthoxycarbonyle, cyano, carbamoyle, N-méthyl-carbamoyle, N-éthylcarbamoyle, N,N-diméthylcarbamoyle, N,N-diéthylcarbamoyle, N-pyrrolidinocarbonyle ou N-pipéridinocarbonyle et R₅ représente un radical alcoxy droit ou ramifié contenant 1 à 6 atomes de carbone.

Plus particulièrement, la présente invention concerne les produits de formule générale (I) dans laquelle Z représente un atome d'hydrogène ou un radical de formule générale (II) dans laquelle R₁ représente un radical benzoyle ou un radical R₂-O-CO-dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5 et R₄ représente un radical hydroxy ou un radical alcoyloxy droit ou ramifié contenant 1 à 6 atomes de carbone ou un radical alcanoyloxy contenant 2 à 6 atomes de carbone et R₅ représente un radical alcoyloxy droit ou ramifié contenant 1 à 6 atomes de carbone.

Plus particulièrement encore, la présente invention concerne les produits de formule générale (I) dans laquelle Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ représente un radical hydroxy ou un radical méthoxy, éthoxy ou propoxy et R₅ représente un radical méthoxy.

Les produits de formule générale (I) dans laquelle Z représente un radical de formule générale (II) présentent des propriétés antitumorales et antileucémiques remarquables.

Des dérivés de taxoïdes présentant la stéréochimie épi du substituant en position 10 (10α) ont été également décrits dans les demandes EP 555 485 et WO 96/03394 ; toutefois les composés 10 α décrits ne portent simultanément en position 7 qu'un radical hydroxyle et aucun enseignement ne ressort de ces demandes concernant la possibilité de substituants éthers sur cette même position 7.

Selon la présente invention, les nouveaux produits de formule générale (I) dans laquelle Z représente un radical de formule générale (II) peuvent être obtenus par estérification d'un produit de formule générale : 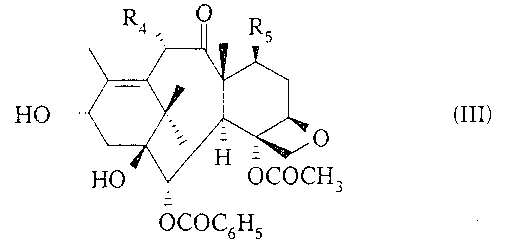 dans laquelle R₄ et R₅ sont définis comme précédemment, au moyen d'un acide de formule générale : 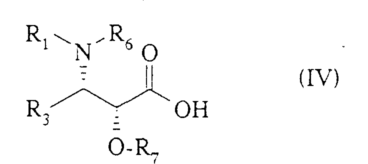 dans laquelle R₁ et R₃ sont définis comme précédemment, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 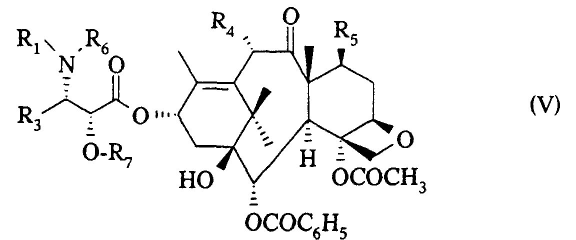 dans laquelle R₁, R₃, R₄, R₅, R₆ et R₇ sont définis comme précédemment, suivi du remplacement des groupements protecteurs représentés par R₇ et/ou R₆ et R₇ par des atomes d'hydrogène.

Pour obtenir un produit de formule générale (I) dans laquelle R₄ représente un radical hydroxy, il est avantageux de protéger la fonction hydroxy en -10 du produit de formule générale (III) préalablement à l'estérification sous forme, par exemple, d'un radical alcoxyacétoxy puis à remplacer le groupement protecteur en -10 par un radical hydroxy au moyen par exemple d'hydrate d'hydrazine et les groupements protecteurs R₇ et/ou R₆ et R₇ par des atomes d'hydrogène.

L'estérification au moyen d'un acide de formule générale (IV) peut être effectuée en présence d'un agent de condensation (carbodiimide, carbonate réactif) et d'un agent d'activation (aminopyridines) dans un solvant organique (éther, ester, cétones, nitriles, hydrocarbures aliphatiques, hydrocarbures aliphatiques halogénés, hydrocarbures aromatiques) à une température comprise entre -10 et 90°C.

L'estérification peut aussi être réalisée en utilisant l'acide de formule générale (IV) sous forme d'anhydride symétrique en opérant en présence d'un agent d'activation (aminopyridines) dans un solvant organique (éthers, esters, cétones, nitriles, hydrocarbures aliphatiques, hydrocarbures aliphatiques halogénés, hydrocarbures aromatiques) à une température comprise entre 0 et 90°C.

L'estérification peut aussi être réalisée en utilisant l'acide de formule générale (IV) sous forme d'halogénure ou sous forme d'anhydride mixte avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

De préférence, R₆ représente un atome d'hydrogène et R₇ représente un groupement protecteur de la fonction hydroxy ou bien R₆ et R₇ forment ensemble un hétérocycle saturé à 5 ou 6 chaînons.

Lorsque R₆ représente un atome d'hydrogène, R₇ représente de préférence un radical méthoxyméthyle, éthoxy-1 éthyle, benzyloxyméthyle, triméthylsilyle, triéthylsilyle, β-triméthylsilyléthoxyméthyle, benzyloxycarbonyle ou tétrahydropyrannyle.

Lorsque R₆ et R₇ forment ensemble un hétérocycle, celui-ci est de préférence un cycle oxazolidine éventuellement mono-substitué ou gem-disubstitué en position -2.

Le remplacement des groupements protecteurs R₇ et/ou R₆ et R₇ par des atomes d'hydrogène peut être effectué, selon leur nature de la manière suivante :
1) lorsque R₆ représente un atome d'hydrogène et R₇ représente un groupement protecteur de la fonction hydroxy, le remplacement des groupements protecteurs par des atomes d'hydrogène s'effectue au moyen d'un acide minéral (acide chlorhydrique, acide sulfurique, acide fluorhydrique) ou organique (acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C, ou au moyen d'une source d'ions fluorures tel qu'un complexe acide fluorhydrique-triéthylamine ou par hydrogénation catalytique,
2) lorsque R₆ et R₇ forment ensemble un hétérocycle saturé à 5 ou 6 chaînons et plus particulièrement un cycle oxazolidine de formule générale : 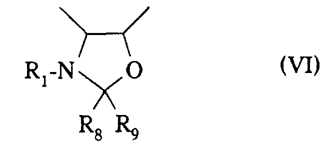 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle tel que trichlorométhyle ou un radical phényle substitué par un radical trihalométhyle tel que trichlorométhyle et R₉ représente un atome d'hydrogène, ou bien R₈ et R₉ forment ensemble avec l'atome de carbone auquel ils sont liés un cycle ayant 4 à 7 chaînons, le remplacement du groupement protecteur formé par R₆ et R₇ par des atomes d'hydrogène peut être effectué, selon les significations de R₁, R₈ et R₉, de la manière suivante :
   a) lorsque R₁ représente un radical tert-butoxycarbonyle, R₈ et R₉, identiques ou différents, représentent un radical alcoyle ou un radical aralcoyle (benzyle) ou aryle (phényle), ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, le traitement de l'ester de formule générale (V) par un acide minéral ou organique éventuellement dans un solvant organique tel qu'un alcool conduit au produit de formule générale : 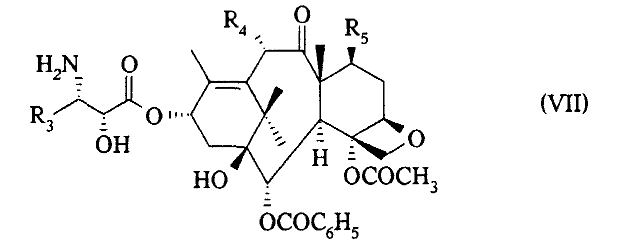 dans laquelle R₃, R₄ et R₅ sont définis comme précédemment, qui est acylé au moyen de chlorure de benzoyle dans lequel le noyau phényle est éventuellemnt substitué, de chlorure de thénoyle, de chlorure de furoyle ou d'un produit de formule générale :

      R₂-O-CO-X (VIII)

      dans laquelle R₂ est défini comme précédemment et X représente un atome d'halogène (fluor, chlore) ou un reste -O-R₂ ou -O-CO-O-R₂, pour obtenir un produit de formule générale (I) dans laquelle Z représente un radical de formule générale (II).
      De préférence, le produit de formule générale (V) est traité par l'acide formique à une température voisine de 20°C pour fournir le produit de formule générale (VII).
      De préférence, l'acylation du produit de formule générale (VII) au moyen d'un chlorure de benzoyle dans lequel le radical phényle est éventuellement substitué, de chlorure de thénoyle ou de chlorure de furoyle ou d'un produit de formule générale (VIII) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.
   b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, le remplacement du groupement protecteur formé par R₆ et R₇ par des atomes d'hydrogène s'effectue en présence d'un acide minéral (acide chlorhydrique, acide sulfurique) ou organique (acide acétique, acide méthanesulfonique, acide trifluorométhanesulfonique, acide p.toluènesulfonique) utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

Selon l'invention, les produits de formule générale (I) dans laquelle R₄ et R₅ sont définis comme précédemment, R4 ne pouvant pas représente un radical hydroxy et Z représente un atome d'hydrogène ou un radical de formule générale (II) peuvent être obtenus par action d'un produit de formule générale :

R'₄-X₁ (IX)

dans laquelle R'₄ est tel que R'₄-O est identique à R₄ défini comme précédemment et X₁ représente un atome d'halogène, sur un produit de formule générale : 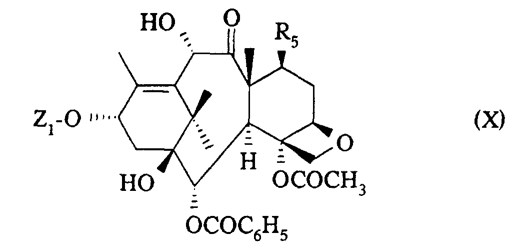 dans laquelle R₅ est défini comme précédemment et Z₁ représente un atome d'hydrogène ou un groupement protecteur de la fonction hydroxy ou un radical de formule générale : 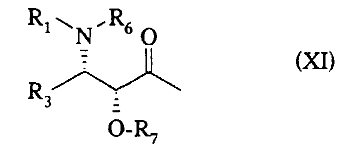 dans laquelle R₁, R₃, R₆ et R₇ sont définis comme précédememnt, suivie éventuellement du remplacement des groupements protecteurs par des atomes d'hydrogène.

Généralement, l'action du produit de formule générale (IX) sur un produit de formule générale (X) est effectuée, après métallation de la fonction hydroxy en position 10 au moyen d'un hydrure de métal alcalin tel que l'hydrure de sodium, un amidure de métal alcalin tel que l'amidure de lithium ou d'un alcoylure de métal alcalin tel que le butyllithium, en opérant dans un solvant organique tel que le diméthylformamide ou le tétrahydrofuarne à une température comprise entre 0 et 50°C.

Lorsque Z₁ représente un groupement protecteur de la fonction hydroxy, celui-ci est de préference un radical silylé, tel qu'un radical trialcoylsilyle, dont le remplacement par un atome d'hydrogène s'effectue au moyen d'un acide tel que l'acide fluorhydrique ou l'acide trifluoroacétique en présence d'une base telle que la triéthylamine ou la pyridine éventuellement substituée par un ou plusieurs radicaux alcoyles contenant 1 à 4 atomes de carbone, éventuellement associée à un solvant organique inerte tel qu'un nitrile comme l'acétonitrile ou un hydrocarbure aliphatique halogéné comme le dichlorométhane à une température comprise entre 0 et 80°C.

Lorsqu Z₁ représente un radical de formule générale (XI), le remplacement des groupements protecteurs R₆ et/ou R₆ et R₇ par des atomes d'hydrogène s'effectue dans les conditions décrites précédemment pour le remplacement des groupements protecteurs du produit de formule générale (V).

Le produit de formule générale (X) peut être obtenu par réduction d'un produit de formule générale : 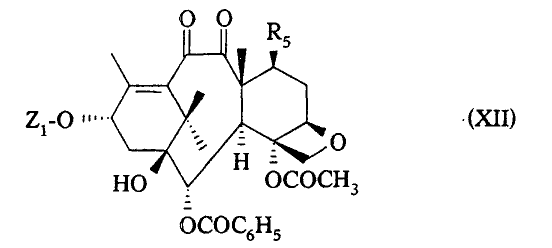 dans laquelle R₅ et Z₁ sont définis comme précédemment.

Généralement, l'agent de réduction est choisi parmi les aluminohydrures ou les hydroborures tels que les hydroborures de métal alcalin ou alcalino-terreux, comme l'hydroborure de sodium, en présence d'un alcool aliphatique contenant 1 à 4 atomes de carbone tel que le méthanol, la réduction étant mise en oeuvre à une température comprise entre 0 et 50°C, de préférence voisine de 20°C.

Le produit de formule générale (XII) peut être obtenu par action d'un agent d'oxydation sur un produit de formule générale : 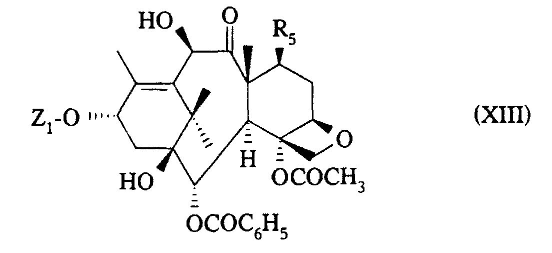 dans laquelle R₅ et Z₁ sont définis comme précédemment.

Généralement, l'agent d'oxydation est choisi parmi les agents qui permettent d'oxyder la fonction alcool secondaire sans toucher au reste de la molécule comme par exemple l'oxygène, le perruthénate d'ammonium, le bioxyde de manganèse, l'acétate de cuivre ou le chlorochromate de pyridinium en opérant dans un solvant organique tel que les hydrocarbures aliphatiques éventuellement halogénés comme le dichlorométhane à une température comprise entre 0 et 50°C, de préférence voisine de 25°C.

Le produit de formule générale (XIII) peut être obtenu par action de l'hydrazine, de préférence sous forme d'hydrate, sur un produit de formule générale : 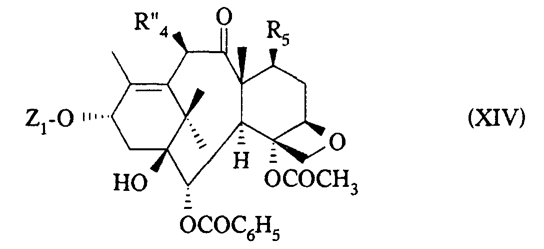 dans laquelle Z₁ et R₅ sont définis comme précédemment et R"₄ représente un radical alcoxyacétoxy ou alcoylthioacétoxy dont la partie alcoyle contient 1 à 4 atomes de carbone.

Généralement, la réaction est effectuée en opérant dans un alcool aliphatique contenant 1 à 4 atomes de carbone comme l'éthanol à une température comprise entre 0 et 50°C.

Le produit de formule générale (XIV) peut être obtenu par action de nickel de Raney activé en présence d'un alcool aliphatique contenant 1 à 3 atomes de carbone sur un produit de formule générale : 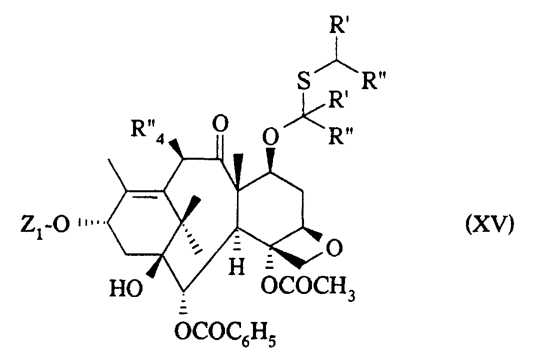 dans laquelle Z₁ et R"₄ sont définis comme précédemment, R' et R", identiques ou différents, représentent un atome d'hydrogène ou un radical alcoyle contenant 1 à 6 atomes de carbone, alcényle contenant 2 à 6 atomes de carbone, alcynyle contenant 2 à 6 atomes de carbone, cycloalcoyle contenant 3 à 6 atomes de carbone ou cycloalcényle contenant 3 à 6 atomes de carbone, ou bien R' et R" forment ensemble avec l'atome de carbone auquel ils sont liés un radical cycloalcoyle contenant 3 à 6 atomes de carbone ou un radical cycloalcényle contenant 4 à 6 atomes de carbone.

Généralement, l'action du nickel de Raney activé en présence de l'alcool aliphatique est effectuée à une température comprise entre -10 et 60°C.

Le produit de formule générale (XV) peut être obtenu par action d'un dialcoylsulfoxyde de formule générale : 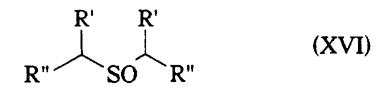 dans laquelle R' et R" sont définis comme précédemment, sur un produit de formule générale : 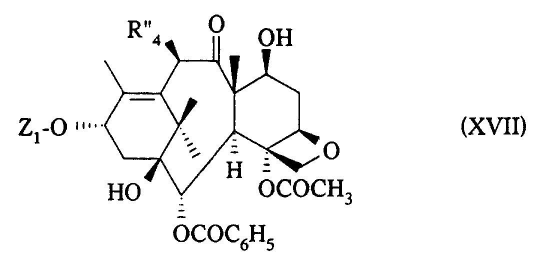 dans laquelle Z₁ et R"₄ sont définis comme précédemment.

Généralement, la réaction du sulfoxyde de formule générale (XVI), de préférence le diméthylsulfoxyde, sur le produit de formule géénrale (XVII) s'effectue en présence d'un mélange d'acide acétique et d'anhydride acétique ou d'un dérivé de l'acide acétique tel qu'un acide halogénoacétique à une température comprise entre 0 et 50°C, de préférence voisine de 25°C.

Le produit de formule générale (XVII) peut être obtenu par action, par exemple d'un complexe triéthylamine-acide fluorhydrique sur un produit de formule générale : 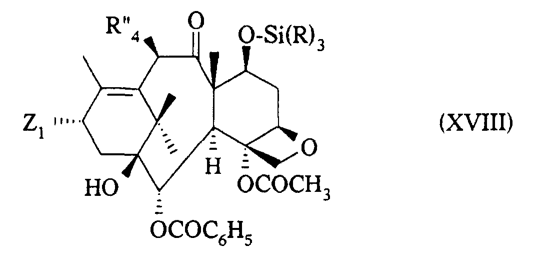 dans laquelle Z₁ et R"₄ sont définis comme précédemment.

Généralement, la réaction est effectuée en opérant dans un solvant organique tel qu'un hydrocarbure aliphatique éventuellement halogéné à un température comprise entre -25 et 25°C.

Le produit de formule générale (XVIII) peut être obtenu dans les conditions décrites dans la demande internationale PCT WO 95/11241.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le taxol et le Taxotère.

In vivo, les produits de formule générale (I) dans laquelle Z représente un radical de formule générale (II) se sont montrés actifs chez la souris greffée par le mélanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug resistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) qui exprime mdr 1.

L'exemple suivant illustre la présente invention.

### EXEMPLE 1

35 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10α méthoxy-7β oxo-9 taxène-11 yle-13α, sont dissous dans 0,74 cm3 d'une solution éthanolique 0,1N d'acide chlorhydrique à 1% d'eau. La solution ainsi obtenue est agitée pendant 2 heures à une température voisine de 20°C puis additionnée de 1 cm3 d'une solution aqueuse saturée d'hydrogénocarbonate de sodium et de 3 cm3 de dichlorométhane. On agite pendant 5 minutes à une température voisine de 20°C et sépare par décantation la phase organique. La phase aqueuse est réextraite par 2 fois 3 cm3 de dichlorométhane. Les phases organiques sont réunies, séchée sur sulfate de magnésium, filtrée et concentrée à sec sous pression réduite (2,7 kPa) à 40°C. On obtient 34 mg d'une meringue blanche que l'on purifie par chromatographie sur gel de silice déposé sur plaques [(gel de 1mm d'épaisseur, 1 plaque de 20 x 20 cm, éluant : dichlorométhane-méthanol (95-5 en volumes)]. Après localisation aux rayons U.V. de la zone correspondant au produit cherché adsorbé, cette zone est grattée et la silice recueillie est lavée sur verre fritté par 10 fois 2 cm3 d'acétate d'éthyle. Les filtrats sont réunis et concentrés à sec sous pression réduite (2,7 kPa) à 20°C. On obtient ainsi 22 mg de tert-butoxycarbonylamino-3 hydroxy-2 phényl-3 propionate-(2R,3S) d'acétoxy-4α benzoyloxy-2α époxy-5β,20 dihydroxy-1β,10α méthoxy-7β oxo-9 taxène-11 yle-13α sous forme d'une meringue blanche dont les caractéristiques sont les suivantes :
- spectre de R.M.N. ¹H (400 MHz ; CDCl₃ ; déplacements chimiques δ en ppm ; constantes de couplage J en Hz) : 1,13 (s, 3H : -CH₃ en 16 ou en 17) ; 1,27 (s, 3H : -CH₃ en 16 ou en 17) ; 1,38 [s, 9H : -C(CH₃)₃] ; 1,72 (s, 3H : -CH₃) ; 1,76 (s, 1H : OH en 1) ; 1,82 et 2,75 (2 mts, 1H chacun : -CH₂- en 6) ; 2,08 (s, 3H : -CH₃) ; 2,28 et 2,35 (2 dd, J = 16 et 9, 1H chacun : -CH₂- en 14) ; 2,40 (s, 3H : -COCH₃) ; 2,71 (d, J = 2, 1H : -OH en 10) ; 3,30 (s, 3H : -OCH₃) ; 3,42 (s large, 1H : -OH en 2') ; 4,20 et 4,32 (2 d, J = 8,5, 1H chacun : -CH₂- en 20) ; 4,27 (d, J = 7,5, 1H: -H en 3) ; 4,31 (mt, 1H : -H en 7) ; 4,64 (mt, 1H: -H en 2') ; 5,02 (d large, J = 10, 1H : -H en 5) ; 5,21 (mt, 1H : -H en 10) ; 5,32 (d large, J = 10, 1H : -H en 3') ; 5,48 (d, J = 10, 1H : -CONH-) ; 5,65 (d, J = 7,5, 1H : -H en 2) ; 6,18 (t large, J = 9, 1H : -H en 13) ; de 7,25 à 7,45 (mt, 5H : -C₆H₅ en 3') ; 7,52 [t, J = 7,5, 2H : -OCOC₆H₅ (-H en 3 et H en 5)] ; 7,63 [t, J = 7,5, 1H : -OCOC₆H₅ (-H en 4)] ; 8,13 [d, J = 7,5, 2H :-OCOC₆H₅ (-H en 2 et H en 6)].

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10α méthoxy-7β oxo-9 taxène-11 yle-13α peut être préparé de la manière suivante :

A une solution de 45 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β dioxo-9,10 taxène-11 yle-13α dans 2 cm3 d'éthanol anhydre, maintenue sous atmosphère d'argon et sous agitation, on ajoute à une température voisine de 20°C, 9 mg de tétrahydruroborate de sodium et maintient le mélange réactionnel sous agitation pendant 1 heure à une température voisine de 20°C puis additionne 1 cm3 d'eau distillée et agite pendant 5 minutes à 20°C. On ajoute 3 cm3 de dichlorométhane et sépare par décantation la phase organique. La phase aqueuse est réextraite par 2 fois 3 cm3 de dichlorométhane. Les phases organiques sont réunies, séchées sur sulfate de magnésium, filtrées sur verre fritté et concentrées à sec sous pression réduite (2,7 kPa) à 40°C. On obtient une meringue blanche que l'on purifie par chromatographie sur gel de silice déposé sur plaque [(gel de 1 mm d'épaisseur, 1 plaque de 20 x 20 cm, éluant : dichlorométhane-méthanol (95-5 en volumes)]. Après localisation aux rayons U.V. de la zone correspondant au produit cherché adsorbé, cette zone est grattée et la silice recueillie est lavée sur verre fritté par 10 fois 2 cm3 d'acétate d'éthyle. Les filtrats sont réunis et concentrés à sec sous pression réduite (2,7 kPa) à 20°C. On obtient ainsi 22,5 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10α méthoxy-7β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β dioxo-9,10 taxène-11 yle-13α peut être préparé de la manière suivante :

A une solution de 100 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10β méthoxy-7β oxo-9 taxène-11 yle-13α dans 5 cm3 de dichlorométhane, maintenue sous atmosphère d'argon et sous agitation, on ajoute successivement, à une température voisine de 20°C, 500 mg de tamis moléculaire 4Å en poudre et 43 mg de chlorochromate de pyridinium. La suspension obtenue est agitée pendant 21 heures à une température voisine de 20°C puis filtrée sur verre fritté garni de célite. Le verre fritté est lavé par 3 fois 5 cm³ de dichlorométhane. Les filtrats sont réunis et additionnés de charbon animal en poudre puis filtrés sur verre fritté et concentrés à sec sous pression réduite (2,7 kPa) à 40°C. On obtient ainsi une meringue jaune que l'on purifie par chromatographie sur gel de silice déposé sur plaque [(gel de 1mm d'épaisseur, 1 plaque de 20 x 20 cm, éluant : dichlorométhane-méthanol (95-5 en volumes)]. Après localisation aux rayons U.V. de la zone correspondant au produit cherché adsorbé, cette zone est grattée et la silice recueillie est lavée sur verre fritté par 10 fois 5 cm3 d'acétate d'éthyle. Les filtrats sont réunis et concentrés à sec sous pression réduite (2,7 kPa) à 20°C. On obtient ainsi 47 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β dioxo-9,10 taxène-11 yle-13α sous forme d'une meringue beige clair.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10β méthoxy-7β oxo-9 taxène-11 yle-13α peut être préparé de la manière suivante :

A une solution de 150 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β méthoxyacétoxy-10β oxo-9 taxène-11 yle-13α dans 4 cm3 d'éthanol anhydre, maintenue sous atmosphère d'argon et sous agitation, on ajoute, goutte à goutte et à une température voisine de 20°C, 0,263 cm3 d'hydrazine mono hydratée. Le milieu réactionnel est maintenu sous agitation pendant 1 heure à une température voisine de 20°C puis versé dans un mélange de 100 cm3 d'acétate d'éthyle et de 50 cm3 d'eau distillée. La phase organique est séparée par décantation et on réextrait la phase aqueuse par 2 fois 50 cm3 d'acétate d'éthyle. Les phases organiques sont réunies lavées par 4 fois 50 cm3 d'eau distillée, séchées sur sulfate de magnésium, filtrées et concentrées à sec sous pression réduite (2,7 kPa) à 40°C. On obtient 180 mg d'une meringue blanche que l'on purifie par chromatographie sur gel de silice déposé sur plaques [(gel de 1mm d'épaisseur, plaques de 20 x 20 cm, éluant : dichlorométhane-méthanol (90-10 en volumes)] par fractions de 90 mg (2 plaques). Après localisation aux rayons U.V. de la zone correspondant au produit cherché adsorbé, cette zone est grattée et la silice recueillie est lavée sur verre fritté par 10 fois 10 cm3 d'acétate d'éthyle. Les filtrats sont réunis et concentrés à sec sous pression réduite (2,7 kPa) à 40°C. On obtient ainsi 113 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10β méthoxy-7β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β méthoxyacétoxy-10β oxo-9 taxène-11 yle-13α peut être préparé de la manière suivante :

A une solution de 1,041 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxyacétoxy-10β méthylthiométhoxy-7β oxo-9 taxène-11 yle-13α dans 100 cm3 d'éthanol anhydre, maintenue sous atmosphère d'argon et sous agitation, on ajoute, à une température voisine de 20°C, 100 cm3 d'une suspension éthanolique de nickel activé selon Raney (obtenue à partir de 80 cm3 de la suspension aqueuse commerciale à environ 50 %, par lavage successifs jusqu'à un pH voisin de 7 par 15 fois 100 cm3 d'eau distillée et par 4 fois 150 cm3 d'éthanol. Le milieu réactionnel est maintenu sous agitation pendant 7 jours à une température voisine de 20°C puis filtré sur verre fritté. Le verre fritté est lavé par 3 fois 100 cm3 d'éthanol, les filtrats sont réunis et concentrés à sec sous pression réduite (2,7 kPa) à 40°C. On obtient 821 mg d'une meringue blanche que l'on purifie par chromatographie sur 75 g de silice (0,063-0,2 mm) contenus dans une colonne de 2,5 cm de diamètre [éluant : dichlorométhane-acétate d'éthyle (90-10 en volumes)] en recueillant des fractions de 5 cm3. Les fractions ne contenant que le produit cherché sont réunies et concentrées à sec sous pression réduite (2,7 kPa) à 40°C. On obtient ainsi 228 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxy-7β méthoxyacétoxy-10β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxyacétoxy-10β méthylthiométhoxy-7β oxo-9 taxène-11 yle-13α peut être préparé de le manière suivante :

A une solution de 5 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,7β méthoxyacétoxy-10β oxo-9 taxène-11 yle-13α dans 165 cm3 de diméthylsulfoxyde anhydre, maintenue sous atmosphère d'argon et sous agitation, on ajoute, à une température voisine de 20°C, 3,35 cm3 d'acide acétique et 11,5 cm3 d'anhydride acétique. Le milieu réactionnel est maintenu sous agitation pendant 3 jours à une température voisine de 20°C puis versé dans 500 cm3 de dichlorométhane. On additionne ensuite sous bonne agitation 100 cm3 d'une solution aqueuse saturée de carbonate de potassium jusqu'à un pH voisin de 7. Après 10 minutes d'agitation, la phase organique est séparée par décantation et on réextrait la phase aqueuse par 2 fois 250 cm3 de dichlorométhane. Les phases organiques sont réunies, lavées par 3 fois 100 cm3 d'eau distillée, séchées sur sulfate de magnésium, filtrées et concentrées à sec sous pression réduite (2,7 kPa) à 40°C. On obtient 9,5 g d'une huile jaune pâle que l'on purifie par chromatographie sur 250 g de silice (0,063-0,4 mm) contenus dans une colonne de 3 cm de diamètre [éluant : dichlorométhane-méthanol (99-1 en volumes)] en recueillant des fractions de 50 cm3. Les fractions ne contenant que le produit cherché sont réunies et concentrées à sec sous pression réduite (2,7 kPa) à 40°C. On obtient ainsi 3,01 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β méthoxyacétoxy-10β méthylthiométhoxy-7β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-2R,4S,5R) d'acétoxy-4α benzoyloxy-2α époxy-5β,20 dihydroxy-1β,7β méthoxyacétoxy-10β oxo-9 taxène-11 yle-13α peut être préparé de la manière suivante :

A une solution de 20 g de tert-butoxycarbonyl-3 (méthoxy-4 phényl)-2 phényl-4 oxazolidine-1,3 carboxylate-5-2R,4S,5R) d'acétoxy-4α benzoyloxy-2α époxy-5β,20 triéthylsilyloxy-7β hydroxy-1β méthoxyacétoxy-10β oxo-9 taxène-11 yle-13α dans 200 cm3 de dichlorométhane anhydre, maintenue sous atmosphère d'argon et sous agitation, on ajoute, goutte à goutte, à une température voisine de 0°C, 220 cm3 du complexe triéthylamine-acide fluorhydrique (1-3 en moles). Le milieu réactionnel est ensuite réchauffé jusqu'à une température voisine de 20°C, maintenu pendant 3 heures à cette température et versé dans 4 litres d'une solution aqueuse saturée d'hydrogénocarbonate de sodium. Le pH du milieu réactionnel est ainsi amené aux environs de 7. Après 10 minutes d'agitation, la phase organique est séparée par décantation et on extrait la phase aqueuse par 2 fois 100 cm3 de dichlorométhane. Les phases organiques sont réunies, lavées par 100 cm3 d'eau distillée, séchées sur sulfate de magnésium, filtrées et concentrées à sec sous pression réduite (2,7 kPa) à 40°C. On obtient ainsi 17,4 g de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,7β méthoxyacétoxy-10β oxo-9 taxène-11 yle-13α sous forme d'une meringue blanche.

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 triéthylsilyloxy-7β hydroxy-1β méthoxyacétoxy-10β oxo-9 taxène-11 yle-13α peut être préparé dans les conditions décrites dans la demande internationale PCT WO 95/11241.

### EXEMPLE 2

Une solution de 40 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β diméthoxy-7β,10α oxo-9 taxène-11 yle-13α dans 0,84 cm3 d'une solution éthanolique 0,1N d'acide chlorhydrique à 1 % d'eau est agitée pendant 2 heures à une température voisine de 20°C puis additionnée de 1 cm3 d'une solution aqueuse saturée d'hydrogénocarbonate de sodium et de 2 cm3 de dichlorométhane. On agite pendant 5 minutes à une température voisine de 20°C et sépare par décantation la phase organique. La phase aqueuse est réextraite par 2 fois 2 cm3 de dichlorométhane. Les phases organiques sont réunies, séchée sur sulfate de magnésium, filtrée et concentrée à sec sous pression réduite (2,7 kPa) à 40°C. On obtient 38 mg d'une meringue blanche que l'on purifie par chromatographie sur gel de silice déposé sur plaque [(gel de 1mm d'épaisseur, 1 plaque de 20 x 20 cm, éluant : dichlorométhane-méthanol (95-5 en volumes)]. Après localisation aux rayons U.V. de la zone correspondant au produit cherché adsorbé, cette zone est grattée et la silice recueillie est lavée sur verre fritté par 10 fois 2 cm3 d'acétate d'éthyle. Les filtrats sont réunis et concentrés à sec sous pression réduite (2,7 kPa) à 20°C. On obtient ainsi 14 mg de tert-butoxycarbonylamino-3 hydroxy-2 phényl-3 propionate-(2R,3S) d'acétoxy-4α benzoyloxy-2α époxy-5β,20 hydroxy-1β diméthoxy-7β,10α oxo-9 taxène-11 yle-13α sous forme d'une meringue blanche dont les caractéristiques sont les suivantes :
- spectre de R.M.N. ¹H (400 MHz ; CDCl₃ ; déplacements chimiques δ en ppm ; constantes de couplage J en Hz) : 1,13 (s, 3H : -CH₃ en 16 ou 1en 7) ; 1,27 (s, 3H : -CH₃ en 16 ou en 17) ; 1,39 [s, 9H : -C(CH₃)₃] ; 1,72 (s, 3H : -CH₃) ; 1,76 (s, 1H : -OH en 1) ; 1,78 et 2,78 (2 mts, 1H chacun : -CH₂- en 6) ; 2,03 (s, 3H : -CH₃) ; 2,27 et 2,37 (2 dd, J = 16 et 9, 1H chacun : -CH₂- en 14) ; 2,38 (s, 3H : -COCH₃) ; 3,28 (s, 3H : -OCH₃) ; 3,42 (s large, 1H : -OH en 2') ; 3,47 (s, 3H : -OCH₃) ; de 4,15 à 4,25 (mt, 2H : -H en 3 et -H en 7) ; 4,18 et 4,32 (2 d, J = 8,5, 1H chacun : -CH₂- en 20) ; 4,52 (s large, 1H : -H en 10) ; 4,64 (mt, 1H : -H en 2') ; 5,01 (d large, J = 10, 1H : -H en 5) ; 5,30 (d large, J = 10, 1H : -H en 3') ; 5,45 (d, J = 10, 1H : -CONH-) ; 5,65 (d, J = 7,5, 1H : -H en 2) ; 6,15 (t large, J = 9, 1H : -H en 13) ; de 7,25 à 7,45 (mt, 5H : -C₆H₅ en 3') ; 7,51 [t, J = 7,5, 2H : -OCOC₆H₅(-H en 3 et H en 5)] ; 7,62 [t, J = 7,5, 1H : -OCOC₆H₅(-H en 4)] ; 8,12 [d, J = 7,5, 2H : -OCOC₆H₅(-H en 2 et H en 6)].

Le tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β diméthoxy-7β,10α oxo-9 taxène-11 yle-13α peut être préparé de la manière suivante

A une solution de 37 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 dihydroxy-1β,10α méthoxy-7β oxo-9 taxène-11 yle-13α dans 0,5 cm3 de diméthylformamide anhydre, maintenue sous atmosphère d'argon et sous agitation, on ajoute à une température voisine de 0°C, 0,5 cm3 d'iodure de méthyle et 3,6 mg d'une dispersion à 50% en poids d'hydrure de sodium dans l'huile minérale. Le milieu réactionnel est maitenu sous agitation pendant 30 minutes à une température voisine de 0°C puis on ajoute 10 cm3 d'acétate d'éthyle et 5 cm3 d'une solution aqueuse saturée de chlorure d'ammonium. Après 5 minutes d'agitation, la phase organique est séparée par décantation et on réextrait la phase aqueuse par 2 fois 5 cm3 d'acétate d'éthyle. Les phases organiques sont réunies, lavées par 2 fois 10 cm3 d'eau distillée, séchées sur sulfate de magnésium, filtrées et concentrées à sec sous pression réduite (2,7 kPa) à 40°C. On obtient une meringue blanche que l'on purifie par chromatographie sur gel de silice déposé sur plaque [(gel de lmm d'épaisseur, 1 plaque de 20 x 20 cm, éluant : dichlorométhane-méthanol (95-5 en volumes)]. Après localisation aux rayons U.V. de la zone correspondant au produit cherché adsorbé, cette zone est grattée et la silice recueillie est lavée sur verre fritté par 10 fois 2 cm3 d'acétate d'éthyle. Les filtrats sont réunis et concentrés à sec sous pression réduite (2,7 kPa) à 20°C. On obtient ainsi 26 mg de tert-butoxycarbonyl-3 (méthoxy-4 phényl)-2 phényl-4 oxazolidine-1,3 carboxylate-5-(2R,4S,5R) d'acétoxy-4α benzoyloxy-2α époxy-5β,20 hydroxy-1β diméthoxy-7β,10α oxo-9 taxène-11 yle-13α sous forme d'une meringue blanche.

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants. Cependant, les compositions peuvent aussi se présenter sous forme de comprimés, de pilules, de poudres ou de granulés administrables par voie orale.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérilisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées telles que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogènes comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

## Revendications

1. Taxoïdes de formule générale : 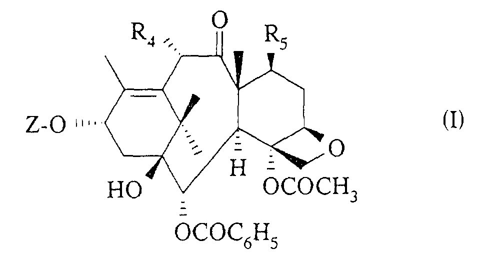 dans laquelle
Z représente un atome d'hydrogène ou un radical de formule générale : 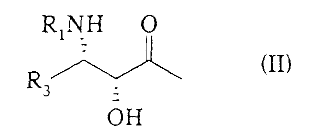 dans laquelle :
R₁ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical R₂-O-CO- dans lequel R₂ représente :
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcovle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
R₄ représente un radical hydroxy ou un radical alcoxy contenant 1 à 6 atomes de carbone en chaîne droite ou ramifiée, alcényloxy contenant 3 à 6 atomes de carbone en chaîne droite ou ramifiée, alcynyloxy contenant 3 à 6 atomes de carbone en chaîne droite ou ramifiée, cycloalcoyloxy contenant 3 à 6 atomes de carbone, cycloalcényloxy contenant 3 à 6 atomes de carbone, alcanoyloxy dont la partie alcanoyle contient 2 à 6 atomes de carbone en chaîne droite ou ramifiée, alcénoyloxy dont la partie alcénoyle contient 3 à 6 atomes de carbone en chaîne droite ou ramifiée, alcynoyloxy dont la partie alcynoyle contient 3 à 6 atomes de carbone en chaîne droite ou ramifiée, alcoxyacétyle dont la partie alcoyle contient 1 à 6 atomes de carbone en chaîne droite ou ramifiée, alcoylthioacétyle dont la partie alcoyle contient 1 à 6 atomes de carbone en chaîne droite ou ramifiée, alcoyloxycarbonyloxy dont la partie alcoyle contient 1 à 6 atomes de carbone en chaîne droite ou ramifiée, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone, ou bien R₄ représente un radical benzoyloxy ou hétérocyclylcarbonyloxy dans lequel la partie hétérocyclique représente un hétérocycle aromatique 5 ou 6 chaînons contenant un ou plusieurs hétéroatomes choisis parmi les atomes d'oxygène, de soufre ou d'azote,
R₅ représente un radical alcoxy contenant 1 à 6 atomes de carbone en chaîne droite ou ramifiée éventuellement substitué par un radical alcoxy contenant 1 à 4 atomes de carbone, alcényloxy contenant 3 à 6 atomes de carbone, alcynyloxy contenant 3 à 6 atomes de carbone, cycloalcoyloxy contenant 3 à 6 atomes de carbone, cycloalcényloxy contenant 3 à 6 atomes de carbone, ces radicaux étant éventuellement substitués par un ou plusieurs atomes d'halogène ou par un radical alcoxy contenant 1 à 4 atomes de carbone, alcoylthio contenant 1 à 4 atomes de carbone, ou un radical carboxy, alcoyloxycarbonyle dont la partie alcoyle contient 1 à 4 atomes de carbone, cyano, carbamoyle, N-alcoylcarbamoyle ou N,N-dialcoylcarbamoyle dont chaque partie alcoyle contient 1 à 4 atomes de carbone ou forme avec l'atome d'azote auquel elle est liée un radical hétérocyclique saturé contenant 5 ou 6 chaînons et éventuellement un second hétéroatome choisi parmi les atomes d'oxygène, de soufre ou d'azote éventuellement substitué par un radical alcoyle contenant 1 à 4 atomes de carbone ou un radical phényle ou un radical phénylalcoyle dont la partie alcoyle contient 1 à 4 atomes de carbone.

2. Taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène et les radicaux alcoyles, alcoxy, dialcoylamino, acylamino, alcoxycarbonylamino ou trifluorométhyle ou un radical furyle-2 ou -3, thiényle-2 ou -3 ou thiazolyle-2, -4 ou -5 et R₄ représente un radical hydroxy ou un radical alcoyloxy droit ou ramifié contenant 1 à 6 atomes de carbone et R₅ représente un radical alcoyloxy droit ou ramifié contenant 1 à 6 atomes de carbone.

3. Taxoïdes selon la revendication 1 pour lesquels Z représente un atome d'hydrogène ou un radical de formule générale (II) dans laquelle R₁ représente un radical benzoyle ou un radical R₂-O-CO- dans lequel R₂ représente un radical tert-butyle et R₃ représente un radical isobutyle, isobutényle, butényle, cyclohexyle, phényle, furyle-2, furyle-3, thiényle-2, thiényle-3, thiazolyle-2, thiazolyle-4 ou thiazolyle-5, R₄ représente un radical hydroxy ou un radical méthoxy et R₅ représente un radical méthoxy.

4. Procédé de préparation d'un produit selon l'une des revendications 1, 2 ou 3 pour lequel Z représente un radical de formule générale (II) caractérisé en ce que l'on estérifie un produit de formule générale : 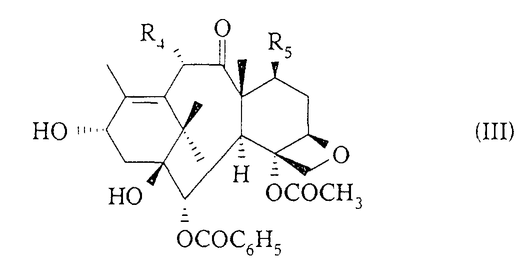 dans laquelle R₄ et R₅ sont définis comme dans l'une des revendications 1, 2 ou 3, R₄ pouvant en outre représenter un groupement protecteur de la fonction hydroxy choisi parmi les radicaux alcoxyacétyle, au moyen d'un acide de formule générale : 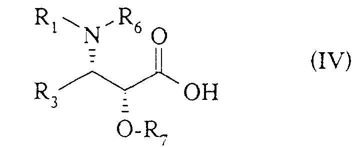 dans laquelle R₁ et R₃ sont définis comme dans l'une des revendications 1, 2 ou 3, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, ou d'un dérivé de cet acide pour obtenir un ester de formule générale : 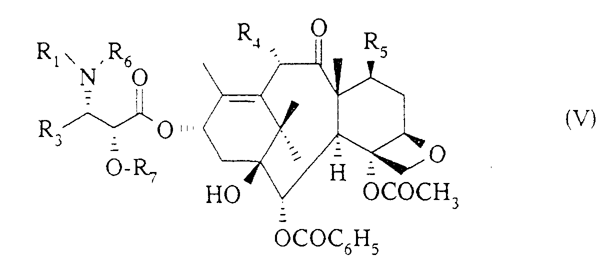 dans laquelle R₁, R₃, R₄, R₅, R₆ et R₇ sont définis comme précédemment, puis remplace les groupements protecteurs représentés par R₇ et/ou R₆ et R₇ par des atomes d'hydrogène et éventuellement le radical R₄ par un radical hydroxy.

5. Procédé selon la revendication 4 caractérisé en ce que l'estérification est effectuée au moyen d'un acide de formule générale (IV) en présence d'un agent de condensation et d'un agent d'activation dans un solvant organique à une température comprise entre -10 et 90°C.

6. Procédé selon la revendication 4 caractérisé en ce que l'estérification est effectuée au moyen d'un acide de formule générale (IV) sous forme d'anhydride symétrique en opérant en présence d'un agent d'activation dans un solvant organique à une température comprise entre 0 et 90°C.

7. Procédé selon la revendication 4 caractérisé en ce que l'estérification est effectuée en utilisant l'acide de formule générale (IV) sous forme d'halogénure ou sous forme d'anhydride mixte avec un acide aliphatique ou aromatique, éventuellement préparé in situ, en présence d'une base en opérant dans un solvant organique à une température comprise entre 0 et 80°C.

8. Procédé selon la revendication 4 caractérisé en ce que l'on remplace les groupements protecteurs R₇ et/ou R₆ et R₇ par des atomes d'hydrogène en opérant, selon leur nature de la manière suivante :
1) lorsque R₆ représente un atome d'hydrogène et R₇ représente un groupement protecteur de la fonction hydroxy, on remplace les groupements protecteurs par des atomes d'hydrogène au moyen d'un acide minéral ou organique utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₆ et R₇ forment ensemble un hétérocycle saturé à 5 ou 6 chaînons de formule générale : 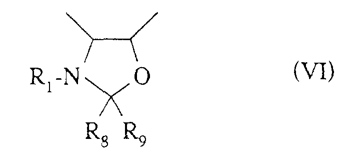 dans laquelle R₁ est défini comme précédemment, R₈ et R₉, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente un radical phényle éventuellement substitué par un ou plusieurs radicaux alcoxy contenant 1 à 4 atomes de carbone, ou un radical aryle représentant un radical phényle éventuellement substitué par un ou plusieurs radicaux alcoxy contenant 1 à 4 atomes de carbone, ou bien R₈ représente un radical alcoxy contenant 1 à 4 atomes de carbone ou un radical trihalométhyle ou un radical phényle substitué par un radical trihalométhyle et R₉ représente un atome d'hydrogène, ou bien R₈ et R₉ forment ensemble avec l'atome de carbone auquel ils sont liés un cycle ayant 4 à 7 chaînons, on remplace le groupement protecteur formé par R₆ et R₇ par des atomes d'hydrogène en opérant, selon les significations de R₁, R₈ et R₉, de la manière suivante :
a) lorsque R₁ représente un radical tert-butoxycarbonyle, R₈ et R₉, identiques ou différents, représentent un radical alcoyle ou un radical aralcoyle ou aryle, ou bien R₈ représente un radical trihalométhyle ou un radical phényle substitué par un radical trihalométhyle, et R₉ représente un atome d'hydrogène, ou bien R₈ et R₉ forment ensemble un cycle ayant de 4 à 7 chaînons, on traite l'ester de formule générale (V) par un acide minéral ou organique éventuellement dans un solvant organique tel qu'un alcool pour obtenir le produit de formule générale : 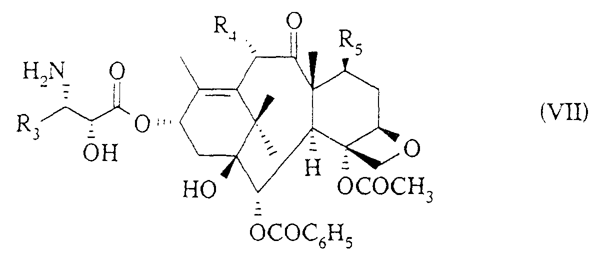 dans laquelle R₃, R₄ et R₅ sont définis comme précédemment, que l'on acyle au moyen de chlorure de benzoyle dans lequel le noyau phényle est éventuellement substitué, de chlorure de thénoyle, de chlorure de furoyle ou d'un produit de formule générale :
R₂-O-CO-X (VIII)
dans laquelle R₂ est défini comme précédemment et X représente un atome d'halogène ou un reste -O-R₂ ou -O-CO-O-R₂, pour obtenir un produit selon l'une des revendications 1, 2 ou 3 pour lequel Z représente un radical de formule générale (II),
b) lorsque R₁ représente un radical benzoyle éventuellement substitué, thénoyle ou furoyle ou un radical R₂O-CO- dans lequel R₂ est défini comme précédemment, R₈ représente un atome d'hydrogène ou un radical alcoxy contenant 1 à 4 atomes de carbone ou un radical phényle substitué par un ou plusieurs radicaux alcoxy contenant 1 à 4 atomes de carbone et R₉ représente un atome d'hydrogène, on remplace le groupement protecteur formé par R₆ et R₇ par des atomes d'hydrogène s'effectue en présence d'un acide minéral ou organique utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C.

9. Procédé de préparation d'un produit selon l'une des revendications 1, 2 ou 3 dans laquelle Z représente un atome d'hydrogène ou un radical de formule générale (II) caractérisé en ce que l'on fait réagir un produit de formule générale :
R'₄-X₁ (IX)
dans laquelle R'₄ est tel que R'₄-O est identique à R₄ défini comme dans l'une des revendications 1, 2 ou 3 et X₁ représente un atome d'halogène sur un produit de formule générale : 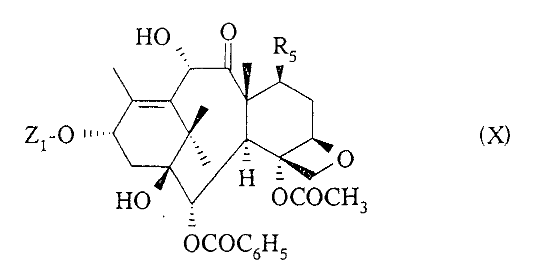 dans laquelle R₅ est défini comme dans l'une des revendications 1, 2 ou 3 et Z₁ représente un atome d'hydrogène ou un groupement protecteur de la fonction hydroxy ou un radical de formule générale : 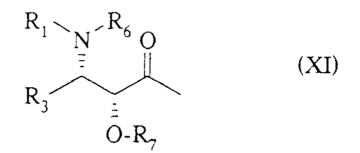 dans laquelle R₁ et R₃ sont définis comme dans l'une des revendications 1, 2 ou 3, ou bien R₆ représente un atome d'hydrogène et R₇ représente un groupement protecteur de la fonction hydroxy, et ou bien R₆ et R₇ forment ensemble un hétérocycle saturé à 5 ou 6 chaînons, puis remplace éventuellement les groupements protecteurs représentés ou portés par Z₁ dans les conditions décrites dans la revendication 8.

10. Procédé selon la revendication 9 caractérisé en ce que l'on fait réagir le produit de formule générale (IX) sur le produit de formule générale (X), après métallation de la fonction hydroxy en position 10 au moyen d'un hydrure de métal alcalin, d'un amidure de métal alcalin ou d'un alcoylure de métal alcalin en opérant dans un solvant organique choisi parmi le diméthylformamide ou le tétrahydrofurane à une température comprise entre 0 et 50°C.

11. Le tert-butoxycarbonylamino-3 hydroxy-2 phényl-3 propionate-(2R,3S) d'acétoxy-4α benzoyloxy-2α époxy-5β,20 dihydroxy-1β,10α méthoxy-7β oxo-9 taxène-11 yle-13α

12. Le tert-butoxycarbonylamino-3 hydroxy-2 phényl-3 propionate-(2R,3S) d'acétoxy-4α benzoyloxy-2α époxy-5β,20 hydroxy-1β diméthoxy-7β,10α oxo-9 taxène-11 yle-13α.

13. Composition pharmaceutique caractérisée en ce qu'elle contient au moins un produit selon l'une des revendications 1, 2 ou 3 pour lequel Z représente un radical de formule générale (II) en association avec un ou plusieurs diluants ou adjuvants pharmaceutiquement acceptables et éventuellement un ou plusieurs composés compatibles et pharmacologiquement actifs.

14. Composition pharmaceutique caractérisée en ce qu'elle contient au moins un produit la revendication 11 en association avec un ou plusieurs diluants ou adjuvants pharmaceutiquement acceptables et éventuellement un ou plusieurs composés compatibles et pharmacologiquement actifs.

15. Composition pharmaceutique caractérisée en ce qu'elle contient au moins un produit selon la revendication 12 en association avec un ou plusieurs diluants ou adjuvants pharmaceutiquement acceptables et éventuellement un ou plusieurs composés compatibles et pharmacologiquement actifs.

## Patentansprüche

1. Taxoide der allgemeinen Formel: 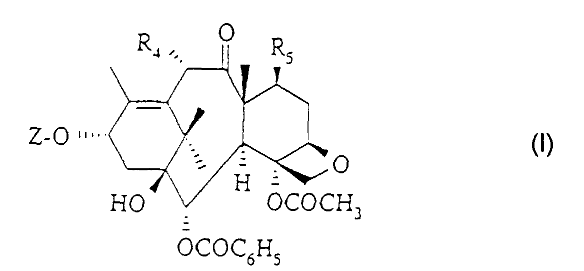 in der
Z ein Wasserstoffatom oder einen Rest der allgemeinen Formel: 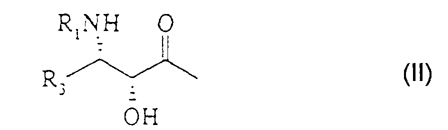 darstellt, in der
R₁ einen Benzoylrest, der gegebenenfalls mit einem oder mehreren gleichen oder verschiedenen Atomen oder Resten substituiert ist, die ausgewählt sind unter den Halogenatomen und den Resten Alkyl mit 1 bis 4 Kohlenstoffatomen, Alkoxy mit 1 bis 4 Kohlenstoffatomen oder Trifluormethyl, Thenoyl oder Furoyl, oder einen Rest R₂-O-CO- darstellt, in dem R₂ darstellt:
- einen Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen, Bicycloalkyl mit 7 bis 10 Kohlenstoffatomen, wobei diese Reste gegebenenfalls mit einem oder mehreren Substituenten substituiert sind, die ausgewählt sind unter den Halogenatomen und den Resten Hydroxy, Alkoxy mit 1 bis 4 Kohlenstoffatomen, Dialkylamino, bei denen jeder Alkylteil 1 bis 4 Kohlenstoffatome enthält, Piperidino, Morpholino, 1-Piperazinyl (gegebenenfalls in Position 4 substituiert mit einem Alkylrest mit 1 bis 4 Kohlenstoffatomen oder mit einem Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl (gegebenenfalls substituiert mit einem oder mehreren Atomen oder Resten, die ausgewählt sind unter den Halogenatomen und den Resten Alkyl mit 1 bis 4 Kohlenstoffatomen oder Alkoxy mit 1 bis 4 Kohlenstoffatomen), Cyano, Carboxy oder Alkoxycarbonyl, bei denen der Alkylteil 1 bis 4 Kohlenstoffatome enthält,
- einen Phenyl- oder α- oder β-Naphthylrest, der gegebenenfalls mit einem oder mehreren Atomen oder Resten substituiert ist, die ausgewählt sind unter den Halogenatomen und den Resten Alkyl mit 1 bis 4 Kohlenstoffatomen oder Alkoxy mit 1 bis 4 Kohlenstoffatomen, oder einen aromatischen heterocyclischen Rest mit 5 Ringgliedern, der vorzugsweise unter den Resten Furyl und Thienyl ausgewählt ist,
- oder einen gesättigten heterocyclischen Rest mit 4 bis 6 Kohlenstoffatomen, der gegebenenfalls mit einem oder mehreren Alkylresten mit 1 bis 4 Kohlenstoffatomen substituiert ist,
R₃ einen geraden oder verzweigten Alkylrest mit 1 bis 8 Kohlenstoffatomen, einen geraden oder verzweigten Alkenylrest mit 2 bis 8 Kohlenstoffatomen, einen geraden oder verzweigten Alkinylrest mit 2 bis 8 Kohlenstoffatomen, einen Cycloalkylrest mit 3 bis 6 Kohlenstoffatomen, einen Phenyl- oder α- oder β-Naphthylrest, gegebenenfalls substituiert mit einem oder mehreren Atomen oder Resten, die ausgewählt sind unter den Halogenatomen und den Resten Alkyl, Alkenyl, Alkinyl, Aryl, Aralkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Alkylcarbamoyl, Dialkylcarbamoyl, Cyano, Nitro und Trifluormethyl, oder einen aromatischen Heterocyclus mit 5 Ringgliedern darstellt, der ein oder mehrere, gleiche oder verschiedene, Heteroatome enthält, die unter den Stickstoff-, Sauerstoff- oder Schwefelatomen ausgewählt sind, und der gegebenenfalls mit einem oder mehreren, gleichen oder verschiedenen, Substituenten substituiert ist, die ausgewählt sind unter den Halogenatomen und den Resten Alkyl, Aryl, Amino, Alkylamino, Dialkylamino, Alkoxycarbonylamino, Acyl, Arylcarbonyl, Cyano, Carboxy, Carbamoyl, Alkylcarbamoyl, Dialkylcarbamoyl oder Alkoxycarbonyl, wobei es sich versteht, dass bei den Substituenten der Phenyl-, α- oder β-Naphthylreste und der aromatischen heterocyclischen Reste die Alkylreste und die Alkylanteile der anderen Reste 1 bis 4 Kohlenstoffatome enthalten und dass die Alkenyl- und Alkinylreste 2 bis 8 Kohlenstoffatome enthalten und dass die Arylreste Phenyl- oder α- oder β-Naphthylreste sind,
R₄ einen Hydroxyrest oder einen Rest Alkoxy mit 1 bis 6 Kohlenstoffatomen in gerader oder verzweigter Kette, Alkenyloxy mit 3 bis 6 Kohlenstoffatomen in gerader oder verzweigter Kette, Alkinyloxy mit 3 bis 6 Kohlenstoffatomen in gerader oder verzweigter Kette, Cycloalkyloxy mit 3 bis 6 Kohlenstoffatomen, Cycloalkenyloxy mit 3 bis 6 Kohlenstoffatomen, Alkanoyloxy, dessen Alkanoylteil 2 bis 6 Kohlenstoffatome in gerader oder verzweigter Kette enthält, Alkenoyloxy, dessen Alkenoylteil 3 bis 6 Kohlenstoffatome in gerader oder verzweigter Kette enthält, Alkinoyloxy, dessen Alkinoylteil 3 bis 6 Kohlenstoffatome in gerader oder verzweigter Kette enthält, Alkoxyacetyl, dessen Alkylteil 1 bis 6 Kohlenstoffatome in gerader oder verzweigter Kette enthält, Alkylthioacetyl, dessen Alkylteil 1 bis 6 Kohlenstoffatome in gerader oder verzweigter Kette enthält, Alkyloxycarbonyloxy, dessen Alkylteil 1 bis 6 Kohlenstoffatome in gerader oder verzweigter Kette enthält, darstellt, wobei diese Reste gegebenenfalls substituiert sind mit einem oder mehreren Halogenatomen oder mit einem Alkoxyrest mit 1 bis 4 Kohlenstoffatomen, Alkylthiorest mit 1 bis 4 Kohlenstoffatomen oder einem Rest Carboxy, Alkyloxycarbonyl, dessen Alkylteil 1 bis 4 Kohlenstoffatome enthält, Cyano, Carbamoyl, N-Alkylcarbamoyl oder N,N-Dialkylcarbamoyl, bei dem jeder Alkylteil 1 bis 4 Kohlenstoffatome enthält oder mit dem Stickstoffatom, an das er gebunden ist, einen gesättigten heterocyclischen Rest bildet, der 5 oder 6 Ringglieder und gegebenenfalls ein zweites Heteroatom enthält, das unter den Sauerstoff-, Schwefel- oder Stickstoffatomen ausgewählt ist, der gegebenenfalls mit einem Alkylrest mit 1 bis 4 Kohlenstoffatomen oder einem Phenylrest oder einem Phenylalkylrest, dessen Alkylteil 1 bis 4 Kohlenstoffatome enthält, substituiert ist, oder R₄ einen Benzoyloxy- oder Heterocyclylcarbonyloxyrest darstellt, bei dem der heterocyclische Teil einen aromatischen Heterocyclus mit 5 oder 6 Ringgliedem, der ein oder mehrere Heteroatome enthält, die unter den Sauerstoff-, Schwefel- oder Stickstoffatomen ausgewählt sind, darstellt,
R₅ einen Rest Alkoxy mit 1 bis 6 Kohlenstoffatomen in gerader oder verzweigter Kette, der gegebenenfalls mit einem Alkoxyrest mit 1 bis 4 Kohlenstoffatomen substituiert ist, Alkenyloxy mit 3 bis 6 Kohlenstoffatomen, Alkinyloxy mit 3 bis 6 Kohlenstoffatomen, Cycloalkyloxy mit 3 bis 6 Kohlenstoffatomen, Cycloalkenyloxy mit 3 bis 6 Kohlenstoffatomen darstellt, wobei diese Reste gegebenenfalls substituiert sind mit einem oder mehreren Halogenatomen oder mit einem Alkoxyrest mit 1 bis 4 Kohlenstoffatomen, Alkylthiorest mit 1 bis 4 Kohlenstoffatomen oder einem Rest Carboxy, Alkyloxycarbonyl, dessen Alkylteil 1 bis 4 Kohlenstoffatome enthält, Cyano, Carbamoyl, N-Alkylcarbamoyl oder N,N- Dialkylcarbamoyl, bei dem jeder Alkylteil 1 bis 4 Kohlenstoffatome enthält oder mit dem Stickstoffatom, an das er gebunden ist, einen gesättigten heterocyclischen Rest bildet, der 5 oder 6 Ringglieder und gegebenenfalls ein zweites Heteroatom enthält, das unter den Sauerstoff-, Schwefel- oder Stickstoffatomen ausgewählt ist, der gegebenenfalls mit einem Alkylrest mit 1 bis 4 Kohlenstoffatomen oder einem Phenylrest oder einem Phenylalkylrest, dessen Alkylteil 1 bis 4 Kohlenstoffatome enthält, substituiert ist.

2. Taxoide gemäß Anspruch 1, für die Z ein Wasserstoffatom oder einen Rest der allgemeinen Formel (II) darstellt, in der R₁ einen Benzoylrest oder einen Rest R₂-O-CO- darstellt, in dem R₂ einen tert.-Butylrest darstellt, und R₃ einen Alkylrest mit 1 bis 6 Kohlenstoffatomen, Alkenylrest mit 2 bis 6 Kohlenstoffatomen, Cycloalkylrest mit 3 bis 6 Kohlenstoffatomen, Phenylrest, gegebenenfalls substituiert mit einem oder mehreren gleichen oder verschiedenen Atomen oder Resten, die ausgewählt sind unter den Halogenatomen und den Resten Alkyl, Alkoxy, Dialkylamino, Acylamino, Alkoxycarbonylamino oder Trifluormethyl, oder einen 2- oder 3-Furyl-, 2- oder 3-Thienyl- oder 2-, 4- oder 5-Thiazolylrest darstellt, und R₄ einen Hydroxyrest oder einen geraden oder verzweigten Alkyloxyrest mit 1 bis 6 Kohlenstoffatomen darstellt, und R₅ einen geraden oder verzweigten Alkyloxyrest mit 1 bis 6 Kohlenstoffatomen darstellt.

3. Taxoide gemäß Anspruch 1, für die Z ein Wasserstoffatom oder einen Rest der allgemeinen Formel (II) darstellt, in der R₁ einen Benzoylrest oder einen Rest R₂-O-CO- darstellt, in dem R₂ einen tert.-Butylrest darstellt, und R₃ einen Rest Isobutyl, Isobutenyl, Butenyl, Cyclohexyl, Phenyl, 2-Furyl, 3-Furyl, 2-Thienyl, 3-Thienyl, 2-Thiazolyl, 4-Thiazolyl oder 5-Thiazolyl darstellt, R₄ einen Hydroxyrest oder einen Methoxyrest darstellt, und R₅ einen Methoxyrest darstellt.

4. Verfahren zur Herstellung eines Produkts gemäß einem der Ansprüche 1, 2 oder 3, für das Z einen Rest der allgemeinen Formel (II) darstellt, dadurch gekennzeichnet, dass man ein Produkt der allgemeinen Formel: 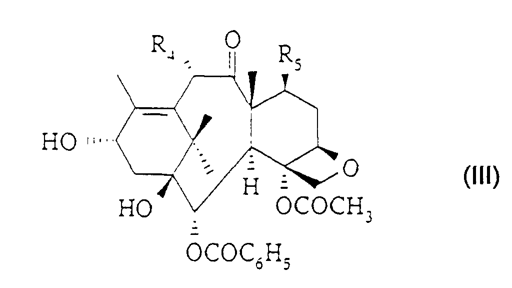 in der R₄ und R₅ wie in einem der Ansprüche 1, 2 oder 3 definiert sind, wobei R₄ außerdem eine Schutzgruppe der Hydroxyfunktion darstellen kann, die unter den Alkoxyacetylresten ausgewählt ist, mittels einer Säure der allgemeinen Formel: 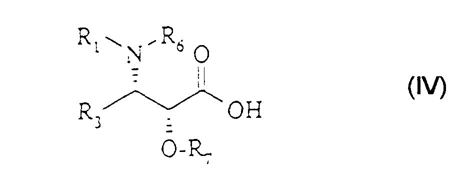 in der R₁ und R₃ wie in einem der Ansprüche 1, 2 oder 3 definiert sind und entweder R₆ ein Wasserstoffatom darstellt und R₇ eine Schutzgruppe für die Hydroxyfunktion darstellt oder R₆ und R₇ zusammen einen gesättigten Heterocyclus mit 5 oder 6 Ringgliedern bilden, oder mittels eines Derivats dieser Säure verestert, um einen Ester der allgemeinen Formel: 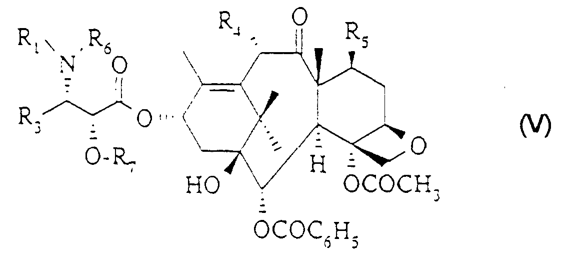 in der R₁, R₃, R₄, R₅, R₆ und R₇ wie zuvor definiert sind, zu erhalten, dann die durch R₇ und/oder R₆ und R₇ dargestellten Schutzgruppen durch Wasserstoffatome und gegebenenfalls den Rest R₄ durch einen Hydroxyrest ersetzt.

5. Verfahren gemäß Anspruch 4, dadurch gekennzeichnet, dass die Veresterung mittels einer Säure der allgemeinen Formel (IV) in Gegenwart eines Kondensationsmittels und eines Aktivierungsmittels in einem organischen Lösungsmittel bei einer Temperatur zwischen -10 und 90 °C ausgeführt wird.

6. Verfahren gemäß Anspruch 4, dadurch gekennzeichnet, dass die Veresterung mittels einer Säure der allgemeinen Formel (IV) in Form des symmetrischen Anhydrids ausgeführt wird, indem man in Gegenwart eines Aktivierungsmittels in einem organischen Lösungsmittel bei einer Temperatur zwischen 0 und 90 °C arbeitet.

7. Verfahren gemäß Anspruch 4, dadurch gekennzeichnet, dass die Veresterung unter Verwendung der Säure der allgemeinen Formel (IV) in Form des Halogenids oder in Form des gemischten Anhydrids mit einer aliphatischen oder aromatischen Säure, das gegebenenfalls in situ hergestellt wird, in Gegenwart einer Base ausgeführt wird, indem man in einem organischen Lösungsmittel bei einer Temperatur zwischen 0 und 80 °C arbeitet.

8. Verfahren gemäß Anspruch 4, dadurch gekennzeichnet, dass man die Schutzgruppen R₇ und/oder R₆ und R₇ durch Wasserstoffatome ersetzt, indem man je nach ihrer Art auf die folgende Weise vorgeht:
1) wenn R₆ ein Wasserstoffatom darstellt und R₇ eine Schutzgruppe der Hydroxyfunktion darstellt, ersetzt man die Schutzgruppen durch Wasserstoffatome mittels einer mineralischen oder organischen Säure, die allein oder im Gemisch verwendet wird, indem man in einem organischen Lösungsmittel, das unter den Alkoholen, den Ethern, den Estern, den aliphatischen Kohlenwasserstoffen, den halogenierten aliphatischen Kohlenwasserstoffen, den aromatischen Kohlenwasserstoffen oder den Nitrilen ausgewählt ist, bei einer Temperatur zwischen -10 und 60 °C arbeitet,
2) wenn R₆ und R₇ zusammen einen gesättigten Heterocyclus mit 5 oder 6 Ringgliedern der allgemeinen Formel: 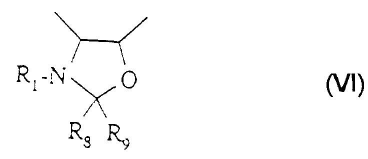 bilden, in der R₁ wie zuvor definiert ist, R₈ und R₉, die gleich oder verschieden sind, ein Wasserstoffatom oder einen Alkylrest mit 1 bis 4 Kohlenstoffatomen oder einen Aralkylrest, dessen Alkylteil 1 bis 4 Kohlenstoffatome enthält und dessen Arylteil einen gegebenenfalls mit einem oder mehreren Alkoxyresten mit 1 bis 4 Kohlenstoffatomen substituierten Phenylrest darstellt, oder einen Arylrest, der einen gegebenenfalls mit einem oder mehreren Alkoxyresten mit 1 bis 4 Kohlenstaffatomen substituierten Phenylrest darstellt, darstellen, oder R₈ einen Alkoxyrest mit 1 bis 4 Kohlenstoffatomen oder einen Trihalogenmethylrest oder einen mit einem Trihalogenmethylrest substituierten Phenylrest darstellt und R₉ ein Wasserstoffatom darstellt, oder R₈ und R₉ zusammen mit dem Kohlenstoffatom, an das sie gebunden sind, einen Ring mit 4 bis 7 Ringgliedern bilden, ersetzt man die von R₆ und R₇ gebildete Schutzgruppe durch Wasserstoffatome, indem man je nach den Bedeutungen von R₁, R₈ und R₉ auf die folgende Weise vorgeht:
a) wenn R₁ einen tert.-Butoxycarbonylrest darstellt, R₈ und R₉, die gleich oder verschieden sind, einen Alkylrest oder einen Aralkyl- oder Arylrest darstellen, oder R₈ einen Trihalogenmethylrest oder einen mit einem Trihalogenmethylrest substituierten Phenylrest darstellt und R₉ ein Wasserstoffatom darstellt, oder R₈ und R₉ zusammen einen Ring mit 4 bis 7 Ringgliedem bilden, behandelt man den Ester der allgemeinen Formel (V) mit einer mineralischen oder organischen Säure gegebenenfalls in einem organischen Lösungsmittel, wie einem Alkohol, um das Produkt der allgemeinen Formel: 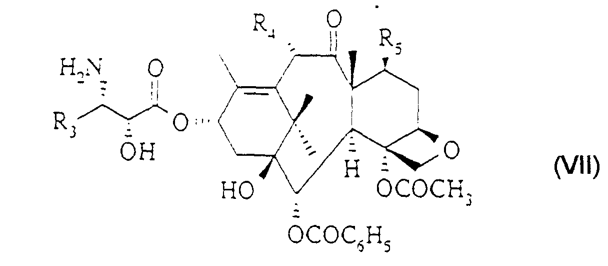 in der R₃, R₄ und R₅ wie zuvor definiert sind, zu erhalten, das man mittels Benzoylchlorid, in dem der Phenylkern gegebenenfalls substituiert ist, Thenoylchlorid, Furoylchlorid oder eines Produkts der allgemeinen Formel:
R₂-O-CO-X (VIII)
in der R₂ wie zuvor definiert ist und X ein Halogenatom oder einen Rest -O-R₂ oder -O-CO-O-R₂ darstellt, acyliert, um ein Produkt gemäß einem der Ansprüche 1, 2 oder 3 zu erhalten, für das Z einen Rest der allgemeinen Formel (II) darstellt,
b) wenn R₁ einen gegebenenfalls substituierten Benzoylrest, Thenoyl- oder Furoylrest oder einen Rest R₂-O-CO-, in dem R₂ wie zuvor definiert ist, darstellt, R₈ ein Wasserstoffatom oder einen Alkoxyrest mit 1 bis 4 Kohlenstoffatomen oder einen mit einem oder mehreren Alkoxyresten mit 1 bis 4 Kohlenstoffatomen substituierten Phenylrest darstellt und R₉ ein Wasserstoffatom darstellt, erfolgt das Ersetzen der von R₆ und R₇ gebildeten Schutzgruppe durch Wasserstoffatome in Gegenwart einer mineralischen oder organischen Säure, die allein oder im Gemisch in stöchiometrischer oder katalytischer Menge verwendet wird, indem man in einem organischen Lösungsmittel, das unter den Alkoholen, den Ethern, den Estern, den aliphatischen Kohlenwasserstoffen, den halogenierten aliphatischen Kohlenwasserstoffen und den aromatischen Kohlenwasserstoffen ausgewählt ist, bei einer Temperatur zwischen -10 und 60 °C arbeitet.

9. Verfahren zur Herstellung eines Produkts gemäß einem der Ansprüche 1, 2 oder 3, in dem Z ein Wasserstoffatom oder einen Rest der allgemeinen Formel (II) darstellt, dadurch gekennzeichnet, dass man ein Produkt der allgemeinen Formel:
R'₄-X₁ (IX)
in der R'₄ derart ist, dass R'₄-O mit R₄, wie in einem der Ansprüche 1, 2 oder 3 definiert, identisch ist und X₁ ein Halogenatom darstellt, mit einem Produkt der allgemeinen Formel: 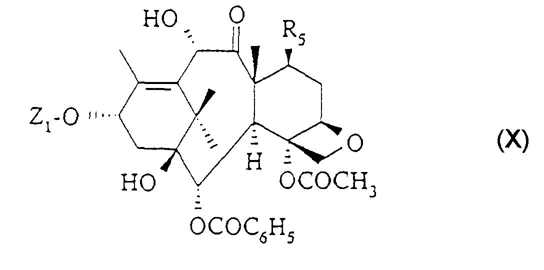 in der R₅ wie in einem der Ansprüche 1, 2 oder 3 definiert ist und Z₁ ein Wasserstoffatom oder eine Schutzgruppe der Hydroxyfunktion cder einen Rest der allgemeinen Formel: 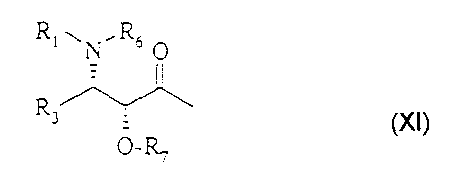 darstellt, in der R₁ und R₃ wie in einem der Ansprüche 1, 2 oder 3 definiert sind, und entweder R₆ ein Wasserstoffatom darstellt und R₇ eine Schutzgruppe der Hydroxyfunktion darstellt, oder R₆ und R₇ zusammen einen gesättigten Heterocyclus mit 5 oder 6 Ringgliedem bilden, umsetzt, dann gegebenenfalls die von Z₁ dargestellten oder getragenen Schutzgruppen unter den in Anspruch 8 beschriebenen Bedingungen ersetzt.

10. Verfahren gemäß Anspruch 9, dadurch gekennzeichnet, dass man das Produkt der allgemeinen Formel (IX) mit einem Produkt der allgemeinen Formel (X) nach Metallsalzbildung an der Hydroxyfunktion in Position 10 mittels eines Alkalimetallhydrids, eines Alkalimetallamids oder eines Alkalimetallalkanids umsetzt, indem man in einem organischen Lösungsmittel, das unter Dimethylformamid oder Tetrahydrofuran ausgewählt ist, bei einer Temperatur zwischen 0 und 50 °C arbeitet.

11. 2R,3S)-3-tert.-Butoxycarbonylamino-2-hydroxy-3-phenyl-propionsäure-4α-acetoxy-2α-benzoyloxy-5β,20-epoxy-1β,10α-dihydroxy-7β-methoxy-9-oxo-11-taxen-13α-yl-ester.

12. 2R,3S)-3-tert.-Butoxycarbonylamino-2-hydroxy-3-phenyl-propionsäure-4α-acetoxy-2α-benzoyloxy-5β,20-epoxy-1β-hydroxy-7β,10α-dimethoxy-9-oxo-11-taxen-13α-yl-ester.

13. Pharmazeutische Zusammensetzung, dadurch gekennzeichnet, dass sie wenigstens ein Produkt gemäß einem der Ansprüche 1, 2 oder 3, für das Z einen Rest der allgemeinen Formel (II) darstellt, in Verbindung mit einem oder mehreren pharmazeutisch zulässigen Verdünnungsmitteln oder Zusatzstoffen und gegebenenfalls einer oder mehreren verträglichen und pharmakologisch wirksamen Verbindungen enthält.

14. Pharmazeutische Zusammensetzung, dadurch gekennzeichnet, dass sie wenigstens ein Produkt gemäß Anspruch 11 in Verbindung mit einem oder mehreren pharmazeutisch zulässigen Verdünnungsmitteln oder Zusatzstoffen und gegebenenfalls einer oder mehreren verträglichen und pharmakologisch wirksamen Verbindungen enthält.

15. Pharmazeutische Zusammensetzung, dadurch gekennzeichnet, dass sie wenigstens ein Produkt gemäß Anspruch 12 in Verbindung mit einem oder mehreren pharmazeutisch zulässigen Verdünnungsmitteln oder Zusatzstoffen und gegebenenfalls einer oder mehreren verträglichen und pharmakologisch wirksamen Verbindungen enthält.

## Claims

1. Taxoids of general formula: 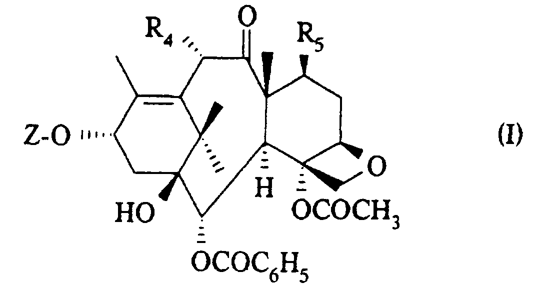 in which
Z represents a hydrogen atom or a radical of general formula: 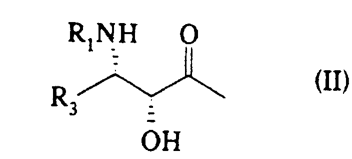 in which:
R₁ represents a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals, thenoyl or furoyl radicals or a radical R₂-O-CO- in which R₂ represents:
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at the 4-position with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,
R₃ represents an unbranched or branched alkyl radical containing 1 to 8 carbon atoms, an unbranched or branched alkenyl radical containing 2 to 8 carbon atoms, an unbranched or branched alkynyl radical containing 2 to 8 carbon atoms, a cycloalkyl radical containing 3 to 6 carbon atoms, a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals, or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals,
R₄ represents a hydroxyl radical or an alkoxy radical containing 1 to 6 carbon atoms in an unbranched or branched chain, an alkenyloxy radical containing 3 to 6 carbon atoms in an unbranched or branched chain, an alkynyloxy radical containing 3 to 6 carbon atoms in an unbranched or branched chain, a cycloalkyloxy radical containing 3 to 6 carbon atoms, a cycloalkenyloxy radical containing 3 to 6 carbon atoms, an alkanoyloxy radical in which the alkanoyl portion contains 2 to 6 carbon atoms in an unbranched or branched chain, an alkenoyloxy radical in which the alkenoyl portion contains 3 to 6 carbon atoms in an unbranched or branched chain, an alkynoyloxy radical in which the alkynoyl portion contains 3 to 6 carbon atoms in an unbranched or branched chain, an alkoxyacetyl radical in which the alkyl portion contains 1 to 6 carbon atoms in an unbranched or branched chain, an alkylthioacetyl radical in which the alkyl portion contains 1 to 6 carbon atoms in an unbranched or branched chain or an alkyloxycarbonyloxy radical in which the alkyl portion contains 1 to 6 carbon atoms in an unbranched or branched chain, these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms, optionally substituted with an alkyl radical containing 1 to 4 carbon atoms or a phenyl radical or a phenylalkyl radical in which the alkyl portion contains 1 to 4 carbon atoms, or alternatively R₄ represents a benzoyloxy radical or a heterocyclylcarbonyloxy radical in which radical the heterocyclic portion represents a 5- or 6-membered aromatic heterocycle containing one or more hetero atoms chosen from oxygen, sulphur and nitrogen atoms,
R₅ represents an alkoxy radical containing 1 to 6 carbon atoms in an unbranched or branched chain optionally substituted with an alkoxy radical containing 1 to 4 carbon atoms, an alkenyloxy radical containing 3 to 6 carbon atoms, an alkynyloxy radical containing 3 to 6 carbon atoms, a cycloalkyloxy radical containing 3 to 6 carbon atoms, a cycloalkenyloxy radical containing 3 to 6 carbon atoms, these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur and nitrogen atoms, optionally substituted with an alkyl radical containing 1 to 4 carbon atoms or a phenyl radical or a phenylalkyl radical in which the alkyl portion contains 1 to 4 carbon atoms.

2. Taxoids according to Claim 1, for which Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms and alkyl, alkoxy, dialkylamino, acylamino, alkoxycarbonylamino and trifluoromethyl radicals, or a 2- or 3-furyl, 2- or 3-thienyl or 2-, 4- or 5-thiazolyl radical, and R₄ represents a hydroxyl radical or an unbranched or branched alkyloxy radical containing 1 to 6 carbon atoms and R₅ represents an unbranched or branched alkyloxy radical containing 1 to 6 carbon atoms.

3. Taxoids according to Claim 1, for which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, R₄ represents a hydroxyl radical or a methoxy radical and R₅ represents a methoxy radical.

4. Process for the preparation of a product according to one of Claims 1, 2 and 3, for which Z represents a radical of general formula (II), characterized in that a product of general formula: 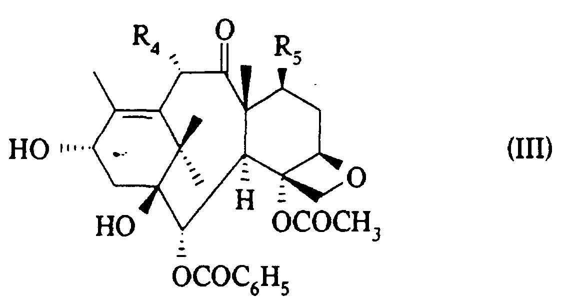 in which R₄ and R₅ are defined as in one of Claims 1, 2 and 3, it being possible for R₄, in addition, to represent a group protecting the hydroxyl function chosen from alkoxyacetyl radicals, is esterified by means of an acid of general formula: 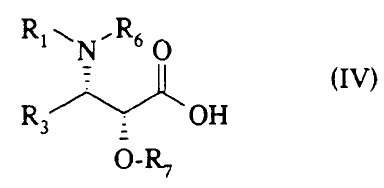 in which R₁ and R₃ are defined as in one of Claims 1, 2 and 3, and either R₆ represents a hydrogen atom and R₇ represents a group protecting the hydroxyl function, or R₆ and R₇ together form a saturated 5- or 6-membered heterocycle, or of a derivative of this acid, to obtain an ester of general formula: 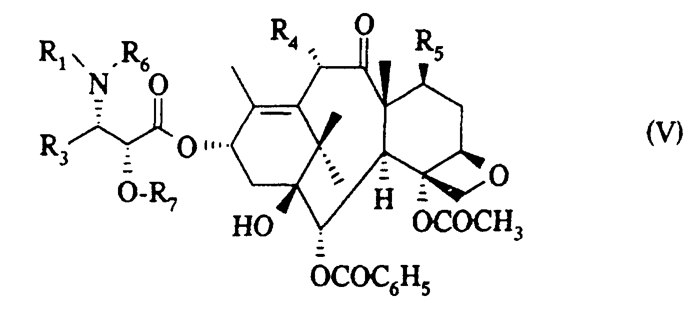 in which R₁, R₃, R₄, R₅, R₆ and R₇ are defined as above, and then the protective groups represented by R₇ and/or R₆ and R₇ are replaced with hydrogen atoms and, optionally, the R₄ radical with a hydroxyl radical.

5. Process according to Claim 4, characterized in that the esterification is carried out by means of an acid of general formula (IV), in the presence of a condensing agent and of an activating agent, in an organic solvent at a temperature of between -10 and 90°C.

6. Process according to Claim 4, characterized in that the esterification is carried out by means of an acid of general formula (IV) in the form of a symmetric anhydride, working in the presence of an activating agent, in an organic solvent at a temperature of between 0 and 90°C.

7. Process according to Claim 4, characterized in that the esterification is carried out using the acid of general formula (IV) in the form of a halide or in the form of a mixed anhydride with an aliphatic or aromatic acid, optionally prepared in situ, in the presence of a base, working in an organic solvent at a temperature of between 0 and 80°C.

8. Process according to Claim 4, characterized in that the protective groups R₇ and/or R₆ and R₇ are replaced with hydrogen atoms, working, according to their nature, in the following manner:
1) when R₆ represents a hydrogen atom and R₇ represents a group protecting the hydroxyl function, the replacement of the protective groups with hydrogen atoms is carried out by means of an inorganic or organic acid, used alone or in the form of a mixture, working in an organic solvent chosen from alcohols, ethers, esters, aliphatic hydrocarbons, halogenated aliphatic hydrocarbons, aromatic hydrocarbons and nitriles, at a temperature of between -10 and 60°C,
2) when R₆ and R₇ together form a saturated 5- or 6-membered heterocycle of general formula: 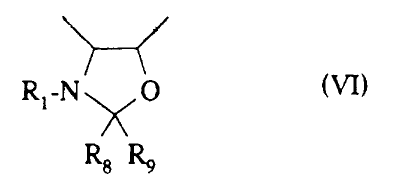 in which R₁ is defined as above, R₈ and R₉, which are identical or different, represent a hydrogen atom or an alkyl radical containing 1 to 4 carbon atoms, or an aralkyl radical in which the alkyl portion contains 1 to 4 carbon atoms and the aryl portion represents a phenyl radical optionally substituted with one or more alkoxy radicals containing 1 to 4 carbon atoms, or an aryl radical representing a phenyl radical optionally substituted with one or more alkoxy radicals containing 1 to 4 carbon atoms, either R₈ represents an alkoxy radical containing 1 to 4 carbon atoms or a trihalomethyl radical or a phenyl radical substituted with a trihalomethyl radical and R₉ represents a hydrogen atom, or R₈ and R₉ together form, with the carbon atom to which they are linked, a ring having 4 to 7 members, the replacement of the protective group formed by R₆ and R₇ with hydrogen atoms is carried out, according to the meanings of R₁, R₈ and R₉, working in the following manner:
a) when R₁ represents a tert-butoxycarbonyl radical, R₈ and R₉, which are identical or different, represent an alkyl radical or an aralkyl or aryl radical, either R₈ represents a trihalomethyl radical or a phenyl radical substituted with a trihalomethyl radical, and R₉ represents a hydrogen atom, or R₈ and R₉ together form a 4- to 7-membered ring, the ester of general formula (V) is treated with an inorganic or organic acid, optionally in an organic solvent such as an alcohol, to give the product of general formula: 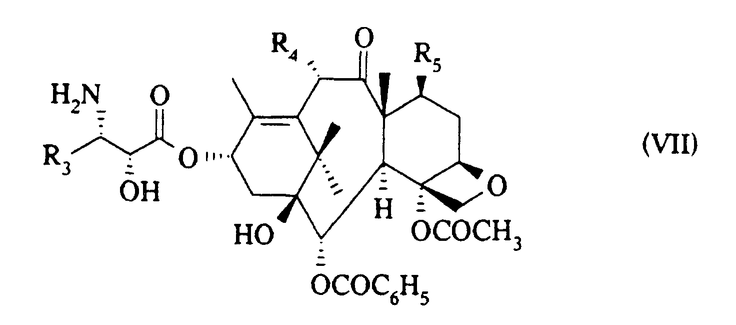 in which R₃, R₄ and R₅ are defined as above, which is acylated by means of benzoyl chloride in which the phenyl ring is optionally substituted, thenoyl chloride, furoyl chloride or a product of general formula:
**R**_{**2**}**-O-CO-X** (VIII)
in which R₂ is defined as above and X represents a halogen atom or a residue -O-R₂ or -O-CO-O-R₂, to obtain a product according to one of Claims 1, 2 and 3 in which Z represents a radical of general formula (II),
b) when R₁ represents an optionally substituted benzoyl radical, a thenoyl or furoyl radical or a radical R₂O-CO- in which R₂ is defined as above, R₈ represents a hydrogen atom or an alkoxy radical containing 1 to 4 carbon atoms or a phenyl radical substituted with one or more alkoxy radicals containing 1 to 4 carbon atoms and R₉ represents a hydrogen atom, the replacement of the protective group formed by R₆ and R₇ with hydrogen atoms is carried out in the presence of an inorganic or organic acid, used alone or in the form of a mixture, in a stoichiometric or catalytic quantity, working in an organic solvent chosen from alcohols, ethers, esters, aliphatic hydrocarbons, halogenated aliphatic hydrocarbons and aromatic hydrocarbons at a temperature of between -10 and 60°C.

9. Process for the preparation of a product according to one of Claims 1, 2 and 3, in which Z represents a hydrogen atom or a radical of general formula (II), characterized in that a product of general formula:
R'₄-X₁ (IX)
in which R'₄ is such that R'₄-O is identical to R₄ defined as in one of claims 1, 2 and 3 and X₁ represents a halogen atom, is reacted with a product of general formula: 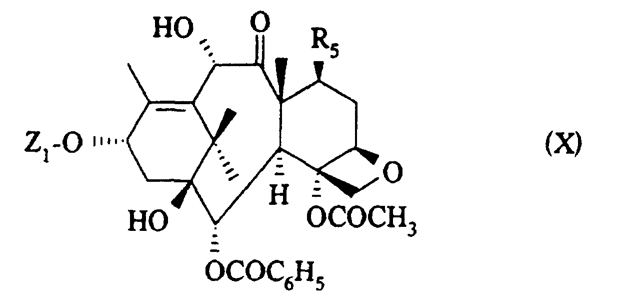 in which R₅ is defined as in one of Claims 1, 2 and 3 and Z₁ represents a hydrogen atom or a group protecting the hydroxyl function or a radical of general formula: 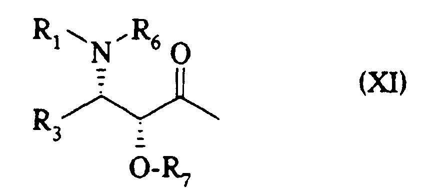 in which R₁ and R₃ are defined as in one of Claims 1, 2 and 3, and either R₆ represents a hydrogen atom and R₇ represents a group protecting the hydroxyl function, or R₆ and R₇ together form a saturated 5- or 6-membered heterocycle, and then the protective groups represented or carried by Z₁ are optionally replaced under the conditions described in Claim 8.

10. Process according to Claim 9, characterized in that the product of general formula (IX) is reacted with the product of general formula (X), after metalation of the hydroxyl function at the 10-position by means of an alkali metal hydride, an alkali metal amide or an alkali metal alkylide, working in an organic solvent chosen from dimethylformamide or tetrahydrofuran, at a temperature of between 0 and 50°C.

11. 4α-Acetoxy-2α-benzoyloxy-5β,20-epoxy-1β,10α-dihydroxy-7β-methoxy-9-oxo-11-taxen-13α-yl (2R,3S)-3-tert-butoxycarbonylamino-2-hydroxy-3-phenylpropionate.

12. 4α-Acetoxy-2α-benzoyloxy-5β,20-epoxy-1β-hydroxy-7β,10α-dimethoxy-9-oxo-11-taxen-13α-yl (2R,3S)-3-tert-butoxycarbonylamino-2-hydroxy-3-phenylpropionate.

13. Pharmaceutical composition characterized in that it contains at least one product according to one of Claims 1, 2 and 3, for which Z represents a radical of general formula (II) in combination with one or more pharmaceutically acceptable diluents or adjuvants and optionally one or more compatible and pharmacologically active compounds.

14. Pharmaceutical composition characterized in that it contains at least one product according to Claim 11 in combination with one or more pharmaceutically acceptable diluents or adjuvants and optionally one or more compatible and pharmacologically active compounds.

15. Pharmaceutical composition characterized in that it contains at least one product according to Claim 12 in combination with one or more pharmaceutically acceptable diluents or adjuvants and optionally one or more compatible and pharmacologically active compounds.
